# EUROPEAN PATENT SPECIFICATION

(11) **EP 3 237 043 B1**
(45) Date of publication and mention of the grant of the patent: **07.08.2019**
(21) Application number: 15810679.9
(22) Date of filing: 17.12.2015
(51) Int. Cl.: A61M 5/162, A61M 5/24, A61M 5/32

(54) **AN INJECTION DEVICE WITH A REMOVABLE CAP**
INJEKTIONSVORRICHTUNG
DISPOSITIF D'INJECTION

(30) Priority: 22.12.2014 EP 14199611; 29.05.2015 EP 15169864
(43) Date of publication of application: 01.11.2017
(73) Proprietor: Novo Nordisk A/S, 2880 Bagsværd (DK)
(72) Inventor: RØRVIG, Simon, 2100 København Ø (DK); JAKOBSEN, Nikolaj Eusebius, 2880 Bagsværd (DK); HANSEN, Steffen, 2880 Bagsværd (DK)
(86) International application number: PCT/EP2015/080216
(87) International publication number: WO 2016/102299

(56) References cited:
- WO-A1-2014/064100
- WO-A1-2014/080020
- WO-A2-03/070296
- US-A1- 2003 144 633

## Description

### THE TECHNICAL FIELD OF THE INVENTION:

The invention relates to an injection device for delivering a plurality of doses of a liquid drug through a number of injections and especially to an injection device wherein the distal end of the housing of the injection device is protected by a removable protective cap between injections. The injection device may be of the automatic type in which a drive member during dose ejection is rotated by a torsion spring to move a piston rod forward inside a cartridge but it may also be of other types not utilising any spring at all or using some other type of spring in a different way.

### DESRIPTION OF RELATED ART:

WO2014/064100 discloses an injection device as defined in the preamble of claim1. US 2003/0144633 disclose a needle assembly in which the distal end of the needle cannula is located inside an air-tight chamber. The air-tight chamber is provided between a needle cover and the needle hub wherein a sealing element also is provided. The sealing element forms a sterility barrier maintaining the distal end of the needle cannula sterile until first use. When the user wants to perform an injection he (or she) mounts the needle hub to an injection device and breaks away the needle cover. The injection device shown is a so-called one-shot device i.e. an injection device which is emptied in one single injection and there after discarded. Since the injection device is discarded after one use only, the sterility barrier is designed such that once it has been broken the needle cover cannot be mounted again to re-establish the air-tight chamber.

A different kind of injection devices are the so-called pre-filled injection devices in which a cartridge permanently embedded in a housing structure contains a predetermined amount of the liquid drug. This liquid drug is injected through a number of injections and when the predetermined amount has been used the user discards the entire injection device. When using such pre-filled injection device, most manufactures recommend using a fresh and preferably sterile needle assembly for each new injection. However, should a user decide to maintain the needle assembly attached to the injection device during storage there is a risk that the liquid drug inside the lumen of the needle cannula will dry out between injections and thereby clog the free passage through the lumen.

The same problem also occurs when using a so-called durable injection device wherein the user is able to change the cartridge. Even if the distal end of the injection device, being pre-filled or durable, is covered by an ordinary removable protective cap between injections clogging due to dried out liquid drug in the lumen of the needle cannula is still a problem.

A simple way of preventing clogging of the lumen of the needle cannula is simply to remove the needle assembly right after an injection and subsequently to mount a new and preferably sterile needle assembly right before the next injection is performed. However, since many people choose to leave the used needle assembly attached to the injection device after an injection has been performed and during storage clogging of the needle cannula is a problem for many people.

In order to see if the lumen of the needle cannula has a free passage, the user usually primes the drug delivery device before use. Such priming also checks if the injection device is operating properly and is referred to as performing an "air shot" or a "flow check". Priming of the injection device is thus usually done to verify that the injection device functions properly in relation to delivering the liquid drug, including ensuring that the needle cannula is not clogged and also to get rid of air potentially present in the liquid drug.

As will be described below, air may become present in the cartridge due to different situations and one or more air gaps may also become present between certain elements of the injection device such as a plunger and a piston rod.

Air in the cartridge may result in a too small a dose of the liquid drug being delivered since some of the ejected volume consists of the trapped air. Air trapped inside the cartridge should therefore always be removed before setting and injection a dose of the liquid drug.

A clogged needle cannula may occur if an injection device with an attached needle cannula is subjected to heat (and especially to dry heat) since parts of the liquid drug located in the lumen of the needle cannula will evaporate leaving residual elements potentially clogging the lumen of the needle cannula partly or fully. "Subjected to heat" also includes ordinary room temperature.

Priming is usually done by holding the injection device in an upright position and expelling small doses of the liquid drug, i.e. performing so-called air-shots, until the air has been ejected and liquid drug sprays from the distal tip of the needle cannula.

It is often recommended to perform priming initially, e.g. a first time a given injection device is to be used at all, since the tolerances during assembling of the injection device and during filling of the cartridge may result in the injection device being initially delivered to the user with a gap between the piston rod and the plunger inside the cartridge. In order to move the piston rod into abutment with the plunger, initial priming is carried out usually following the same procedure as when performing normal air-shots i.e. performing a number of small ejections

Usually a piston rod foot or piston washer is provided between the piston rod and the plunger of the cartridge to distribute the force from the piston rod to a greater area of the plunger. Such piston rod foot or washer is usually either a separate element loosely provided between the piston rod and the plunger or it is coupled to the piston rod e.g. formed uniform with the piston rod. This piston rod foot or washer is when priming brought into engagement with both the plunger and the piston rod through a number of air-shots.

It is often also recommended to perform priming before each time the user is about to perform an injection. However, it is a concern that not all users properly prime their injection devices every time they should and hence do not fully follow the instructions for use, and it is also known that not all users are fully aware of the reasons and importance of priming their injection device.

Furthermore, some injection device comprise a needle assembly or the like that is detachable from the injection device. At least for some of such injection devices, the user should remove the needle assembly after use and attach a new one before the next use. However, not all users remove the needle assembly after use, but rather keep it mounted on the injection device. Further in some injection devices the needle cannula is permanently connected to the housing assembly of the injection device and the user performs multiple injections through the same permanently mounted needle cannula.

As mentioned, air may become present inside the cartridge in different situations when the needle cannula is maintained inserted into the cartridge between injections. Should the injection device e.g. be subjected to temperature changes air can be sucked into the cartridge through the lumen of the attached needle cannula.

It is not uncommon that a user stores the injection device with an attached needle assembly e.g. in the refrigerator to keep the liquid drug cold, even if that is actually not necessary. In such a case, the liquid drug in the cartridge will contract due to the lower temperature e.g. in the refrigerator, which will create a vacuum which will actively cause outside air to be sucked into the cartridge resulting in air-bubbles in the liquid drug in the cartridge. This will also happen although a protective cap is mounted onto the housing assembly since the usual protective caps are not completely air tight.

When an injection device is subjected to a higher temperature, e.g. by being removed from a refrigerator, the liquid drug in the cartridge will expand and it will likely leak out of the needle cannula. If then followed by a subsequent cooling, additional air will be sucked into the cartridge resulting in even more air-bubbles in the liquid drug.

These adverse effects will be increased by repeated temperature increases and decreases as happening when a user repeatedly uses an injection device and stores it with the needle cannula attached e.g. in a refrigerator between injections.

Furthermore, when the liquid drug expands or contracts due to temperature changes, the plunger of the injection device also moves accordingly in the proximal or distal direction. This may cause the plunger inside the cartridge to move out of its abutment with the piston rod and thus create an air gap between the plunger and the piston rod (or the piston rod foot if such is used).

It would be a benefit to have an injection device that does not require priming or require priming at least to a lesser extent.

Several injection devices have been proposed that aims at addressing the need of having to prime the injection device. However, many of these solutions add complexity and costs to the injection device and some may also add additional length to the injection device.

Furthermore, in order to reduce the waste of the liquid drug contained in the injection device and to simplify the use of the injection device a reduction or an avoidance of both initial priming and air-shots between injections is to be preferred.

It would also be a benefit to have an injection device that avoids or at least reduces the likelihood of clogging in the lumen of the needle cannula attached to an injection device.

### DESCRIPTION OF THE INVENTION:

It is an object to alleviate at least one or more of the above mentioned drawbacks at least to an extent.

Furthermore, it is an object to provide a mechanism in which initial priming and/or general priming i.e. air-shots between injections can, at least partly, but preferably fully, be avoided.

Besides avoiding air to pass into the cartridge during storage it is also an object of the present invention to prevent clogging of the lumen of the needle cannula should the user (or the manufacture) maintain the needle cannula on the injection device between injections.

The invention is defined in claim 1. Accordingly, in one aspect the present invention relates to an injection device for injecting a plurality of doses of a liquid drug through a number of injections. The injection device comprises:
- A housing structure or assembly which supports or holds a cartridge. The cartridge is either permanently embedded in the housing or exchangeable provided in the housing. The housing assembly however only needs to support the cartridge when in use i.e. when performing an injection. When supporting a cartridge, the cartridge has a distal end sealed by a pierceable septum and a proximal end sealed by a movable plunger. The septum and plunger together with the wall of the cartridge defines an interior containing the liquid drug.
- A piston rod moving the movable plunger, preferably in the distal direction, inside the cartridge.
- A protective cap assembly removable coupled to the housing assembly and surrounding at least a distal end of the housing assembly between injections. The removable protective cap assembly is preferably press fitted to the housing when mounted but could be removable attached to the housing structure using any kind of known attachment means e.g. a bayonet coupling or the like.
- A needle mount securing a needle cannula at least in use, or alternatively a needle mount for mounting there upon a needle cannula. It is to be understood that the presence of a needle cannula is only needed during injection. The needle cannula is e.g. secured to the needle mount by gluing or the like. Further, the needle mount can be a permanent part of the housing assembly or it can be detachable from the housing assembly thus allowing replacement. The needle cannula has a proximal end in liquid communication with the interior of the cartridge and a distal end with a distal tip for penetrating through the skin of a user such that the liquid drug is able to flow from the interior of the cartridge through the lumen of the needle cannula and into the subcutaneous layer of the users body.

When the user mounts the removable protective cap assembly it surrounds at least the distal tip of the needle cannula between injections. Further an air-tight seal is provided between the removable protective cap assembly and the housing assembly such that an air-tight chamber is established surrounding at least the distal tip of the needle cannula when the removable protective cap assembly is fitted to the distal end of the housing assembly.

An air-tight seal is any kind of seal that prevents the passage of air to such extend that the liquid drug in the lumen of the needle cannula is prevented from evaporating at normal room temperature i.e. 20-25 degrees Celsius in the normal time elapsing between two subsequent injections. In one example the liquid drug could be any kind of basal insulin which is usually injected once every 24 hours why in this example the air-tight seal must keep the chamber air-tight chamber air-tight for at least 24 hours thus preventing clogging in the time elapsing between two injections. The generally know protective caps as normally used for injection devices currently on the market does not fall under this definition and is generally not recognized as being air-tight. If a user stores a well-known injection device with the needle cannula mounted thereon and concealed only by the standard protective cap, the liquid drug will henceforth evaporate and the lumen of the needle cannula will clog.

Since the distal tip of the needle cannula is maintained in an air-tight chamber between injections, the liquid drug in the lumen of the cartridge is prevented from evaporating. The air in the chamber is quickly humidified by evaporating fumes from the cartridge, and the relatively high humidity around the distal tip of the needle cannula has been shown to actively prevent clogging by preventing further evaporation.

Further, should the liquid drug in the cartridge be subjected to a temperature decrease, the liquid drug will contract. However, the air contained inside the air-tight chamber will contract more as air or gas generally contracts more than a liquid. This will create a situation in which the liquid drug will be dragged from the cartridge towards the air-tight chamber. This will efficiently avoided that air bubbles are introduced into the liquid drug in the cartridge or at least that the likelihood thereof is reduced. Contraction of the liquid inside the cartridge will simultaneously move the plunger distally inside the cartridge, however, the contraction of the air inside the air-tight chamber is still able to move liquid through the lumen of the needle cannula.

As the liquid drug is prevented from evaporating and, depending on the temperature changes, the liquid drug is moved from the cartridge and towards or even into the air-tight chamber priming between injections can be effectively prevented.

The needle cannula is in one example mounted in a needle hub which is attachable to the needle mount provided at the housing assembly. The needle mount carries connection means preferably in the form of a bayonet mount or a threaded mount or any combination thereof. The needle cannula could however also be permanently mounted to the housing assembly preferably by making the needle mount carrying the needle cannula a part of the housing assembly.

The housing assembly is preferably provided with a radially pointing flange which is located at the distal end of the housing assembly and preferably points towards a centre line of the injection device. This radial flange abuts the distal end of the cartridge at its proximal surface and abuts the hub of the needle assembly on its distal surface.

The radial flange is made relatively thin and is preferably moulded such that the flange points a few degrees in the proximal direction i.e. towards the cartridge. Further, the cartridge is pressed distally in the housing assembly during assembly such that the distal end of the cartridge is forced into abutment with the radial flange which due to its tilting direction forms an effective seal to the cartridge. Oppositely the radial flange seals against the needle hub.

As mentioned, the air-tight seal is operational provided between the removable protective cap assembly and the housing assembly. However, when a detachable needle hub is mounted on the needle mount, the air-tight seal can alternatively be provided between the removable protective cap assembly and the needle hub mounted on the needle mount as will be explained later.

In one embodiment of the invention, the air-tight seal is provided on an inner surface of the removable protective cap assembly. The air-tight seal can either be provided as a separate element e.g. an O-ring or the like that is glued or otherwise secured to the removable protective cap assembly. Alternatively the air-tight seal can be provided as an integral part of the removable protective cap assembly. The air-tight seal could e.g. be moulded together with the removable protective cap assembly in a 2K moulding.

When the air-tight seal is provided on an inner surface of the protective cap assembly it is preferably carried on an inner sealing element being a part of the cap assembly. In one example, the inner sealing element is connected to the outer cap element via a resiliency which could be any kind of resilient element such as compression spring operating between the outer cap element and the inner sealing element. This resiliency urges the inner sealing element in the proximal direction such that when the removable protective cap is mounted to the distal end of the housing assembly, the resiliency forces the inner sealing element including the air-tight seal against the housing assembly thereby enhancing the sealing effect.

The resiliency in form of a resilient element could alternatively be moulded as an integral part of either the inner sealing element or the outer cap element, however, any known metallic compression spring could also be used.

In a second embodiment of the invention, the air-tight seal is operational provided on an external surface of the housing assembly and preferably in the proximity of the needle mount. As an alternative to physically providing the air-tight seal on the removable protective cap assembly, the air-tigh seal can be physically provided on the housing assembly. The air-tight seal can be embodied either as a separate element attached to the housing assembly or as an integral part of the housing assembly, e.g. made integral by a 2K moulding.

In both the first and the second embodiment, the air-tight seal is preferably made from a thermoplastic rubber material such as a well-known TPE (Thermo Plastic Elastomer).

The air-tight seal is preferably radially expandable to cover a larger outer diameter when the needle hub is attached to the needle mount. In one example, the air-tight seal has a first position where the air-tight chamber is not established and a second position where the air-tight chamber is established, and wherein the air-tight seal is brought from the first position to the second position in response to a needle assembly being attached to the injection device.

This has the advantage that the air-tight seal may be in its first rested or relaxed position during storage at the manufacturer or at the user. In this way, relaxation of the air-tight seal, e.g. being a rubber material or the like, is prevented even when being stored for a relatively long time. Furthermore, the use or function of the air-tight seal is activated at the specific time when it is needed in a very user convenient and expedient manner simply by attaching the needle assembly to the needle mount of the housing assembly, which the user would do any way before regular use.

In a further example the injection device further comprises a ratchet element limiting movement of the piston rod to the distal direction. This has the effect that should the liquid drug inside the cartridge be exposed to an increased temperature, the liquid drug inside the cartridge would expand. Such expansion could potentially move the plunger proximally inside the cartridge which could draw air into the interior of the cartridge. This is however effectively prevented by preventing the piston rod and henceforth the plunger from movement in the proximal direction.

Thus only allowing movement of the plunger in the distal direction creates a situation in which the liquid drug will be drawn from the cartridge and distally through the lumen of the needle cannula if the injection device is subjected to a temperature decrease as explained above and at the same time prevent air from entering into the cartridge should the injection device be subjected to a temperature increase.

Therefore preventing axial movement of the plunger in the proximal direction but allowing movement in the distal direction in combination with the above air-tight chamber further limits the need for priming of the injection device between injections.

Preventing the piston rod from axial movement in the proximal direction can be done in numerous ways. In one example a one-way clutch engaging the piston rod can be provided. Such one-way clutch could e.g. be keyed to piston rod and having a toothed one-way engagement with the housing structure such that the piston rod is allowed only to rotate in one rotational direction being the direction moving the piston rod in the distal direction. However, the piston rod need not rotate at all in which case the one-way clutch could e.g. be threaded to the piston rod such that rotation of the clutch in one direction would move the piston rod forward without rotation. In the opposite rotational direction the clutch could e.g. be coupled to the housing assembly via a one-way ratchet preventing rotation in the direction that would move the piston rod in the proximal direction. The clutch could further be combined with the drive element driving the piston rod or the clutch could in fact be the drive element.

In one example, the piston rod and the movable plunger of the cartridge are axially coupled together such that the piston rod and the movable plunger of the cartridge move together at least in the axially direction. The coupling of the piston rod and the plunger could in one example be via an intermediate piston rod foot which is fixed both to the movable plunger and to the piston rod. If the piston rod is rotatable during dose expelling, the coupling between the piston rod and the intermediate piston rod foot is preferably a coupling allowing such rotation. In this example, the issues of air gaps between the plunger and the piston rod foot and/or between the piston rod foot and the piston rod are addressed since they are now axially connected and will follow each other rigidly whereby the air gaps cannot happen anymore.

In a further example, the injection device further comprises an additional air-tight seal located between an internal surface of the housing assembly and the outer surface of the cartridge. This additional air-tight seal provides an air-tight chamber even for injection device designs that otherwise allows a flow of air between the inner surface of the housing and the outer surface of the cartridge.

The removable protective cap assembly could further be provided with a one-way valve that would allow air to flow in one direction only. When the user re-mounts the protective cap assembly to the housing assembly the protective cap assembly slides a distance relatively to the housing and thus to the air-tight sealing in case the air-tight seal is provided on the housing structure, which would cause air to be trapped and compressed inside the interior of the movable protective cap. Due to this compression there is a risk that the trapped air will be pressed through the longitudinal lumen of the needle cannula and into the interior of cartridge which is highly unwanted since it creates air bubbles in the liquid drug.

To prevent this and to allow the trapped air to escape during re-mounting of the cap assembly, the interior of the protective cap assembly is preferably provided with a one-way valve which can be embodied in a number of different ways. The one-way valve could in one example be formed by as a rubber insert moulded on the inside of the outer cap element and which rubber insert distally terminates in a closed tip provided with a longitudinal slit through which the trapped air can escape. Thus when the protective cap assembly slides proximally on the housing assembly and on the air-tight seal, the trapped air can escape through this longitudinal slit.

Although the use of such one-way valve is primarily intended to be used in embodiments wherein the air-tight seal is provided on the housing either as a separate seal or as an integrated seal this does not rule out that such one-way valve can be used in other embodiments of the invention.

As previously referred to, the air-tight seal is established between the removable protective cap assembly and the housing assembly and is preferably provided either on the cap assembly or on the housing assembly. However, if a needle assembly is attached to the needle mount of the housing assembly, the air-tight seal can alternatively be provided between the removable protective cap assembly and the needle hub attached to the needle mount provided distally at the housing assembly. These further examples of the present invention can be expressed as follows.
Example A) An injection device for injecting a plurality of doses of a liquid drug through a number of injections, the injection device comprising:
   a housing assembly supporting a cartridge, at least in use, which cartridge has a distal end sealed by a pierceable septum and a proximal end sealed by a movable plunger defining an interior containing the liquid drug,
   a piston rod for moving the movable plunger distally inside the cartridge,
   a removable protective cap assembly removable coupled to the housing assembly and surrounding at least a distal end of the housing assembly between injections, and
   a needle mount, at least in use, carrying a needle assembly comprising a needle cannula secured in a needle hub which needle cannula has a proximal end in liquid communication with the interior of the cartridge and a distal end with a distal tip for penetrating the skin of a user.

   The injection device is further characterized in that the removable protective cap assembly surrounds at least the distal tip of the needle cannula between injections, and an air-tight seal is provided between the removable protective cap assembly and the needle hub mounted to the needle mount such that an air-tight chamber is established surrounding at least the distal tip of the needle cannula when the removable protective cap assembly is fitted to the distal end of the housing assembly.
   The air-tight seal is in the above example intended to be provided on the removable protective cap assembly but could alternatively be provided on an outer surface of the needle hub. This further embodiment could further be combined with any of the below examples.
Example B) An injection device according to example A), wherein the housing assembly) distally is provided with a radial flange against which flange both the needle hub and the cartridge abuts.
Example C) An injection device according to any of the examples A) or B), wherein the air-tight seal is provided on an inner surface of the removable protective cap assembly.
Example D) An injection device according to any of the examples A), B) or C), wherein the removable protective cap assembly comprises an inner sealing element and an outer cap element.
Example E) An injection device according to example D), wherein the inner sealing element carries the air-tight seal.
Example F) An injection device according to any of the examples D) or E), wherein the inner sealing element is coupled to the outer cap element via a resiliency such as a resilient element providing the sealing element with an axial force relatively to the outer cap element.
Example G) An injection device according to any of examples A) to F), wherein the injection device further comprises a ratchet element limiting axial movement of the piston rod (280) to the distal direction.
Example H) An injection device according to any of the examples A) to G), wherein the piston rod and the movable plunger are axially coupled together.
Example I) An injection device according to example H), wherein the piston rod and the movable plunger are axially coupled via an intermediate piston rod foot which is fixed to the movable plunger and rotatably connected with the piston rod.
Example J) An injection device according to any of the examples A) to I), wherein the injection device further comprises an additional air-tight seal located between an internal surface of the housing assembly and an outer surface of the cartridge.
Example K) An injection device according to any of the examples A) to J), wherein the protective cap assembly is provided with a one-way valve.

### DEFINITIONS:

An **"injection pen"** is typically an injection apparatus having an oblong or elongated shape somewhat like a pen for writing. Although such pens usually have a tubular cross-section, they could easily have a different cross-section such as triangular, rectangular or square or any variation around these geometries.
The term **"Needle Cannula"** is used to describe the actual conduit performing the penetration of the skin during injection. A needle cannula is usually made from a metallic material such as e.g. stainless steel and connected to a hub to form a complete injection needle also often referred to as a **"needle assembly".** A needle cannula could however also be made from a polymeric material or a glass material. The hub also carries the connecting means for connecting the needle assembly to an injection apparatus and is usually moulded from a suitable thermoplastic material. The **"connection means"** could as examples be a luer coupling, a bayonet coupling, a threaded connection or any combination thereof e.g. a combination as described in EP 1,536,854.
As used herein, the term **"drug"** is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.
**"Cartridge"** is the term used to describe the container actually containing the drug. Cartridges are usually made from glass but could also be moulded from any suitable polymer. A cartridge or ampoule is preferably sealed at one end by a pierceable membrane referred to as the **"septum"** which can be pierced e.g. by the non-patient end of a needle cannula. Such septum is usually self-sealing which means that the opening created during penetration seals automatically by the inherent resiliency once the needle cannula is removed from the septum. The opposite end is typically closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. However, any kind of container - rigid or flexible - can be used to contain the drug.
Since a cartridge usually has a narrower distal neck portion into which the plunger cannot be moved not all of the liquid drug contained inside the cartridge can actually be expelled. The term **"initial quantum"** or **"substantially used"** therefore refers to the injectable content contained in the cartridge and thus not necessarily to the entire content.
By the term **"Pre-filled"** injection device is meant an injection device in which the cartridge containing the liquid drug is permanently embedded in the injection device such that it cannot be removed without permanent destruction of the injection device. Once the pre-filled amount of liquid drug in the cartridge is used, the user normally discards the entire injection device. This is in opposition to a **"Durable"** injection device in which the user can himself change the cartridge containing the liquid drug whenever it is empty. Pre-filled injection devices are usually sold in packages containing more than one injection device whereas durable injection devices are usually sold one at a time. When using pre-filled injection devices an average user might require as many as 50 to 100 injection devices per year whereas when using durable injection devices one single injection device could last for several years, however, the average user would require 50 to 100 new cartridges per year.
Using the term **"Automatic"** in conjunction with injection device means that, the injection device is able to perform the injection without the user of the injection device delivering the force needed to expel the drug during dosing. The force is typically delivered - automatically - by an electric motor or by a spring drive. The spring for the spring drive is usually strained by the user during dose setting, however, such springs are usually prestrained in order to avoid problems of delivering very small doses. Alternatively, the spring can be fully preloaded by the manufacturer with a preload sufficient to empty the entire drug cartridge though a number of doses. Typically, the user activates a latch mechanism e.g. in the form of a button on, e.g. on the proximal end, of the injection device to release - fully or partially - the force accumulated in the spring when carrying out the injection.
The term **"Permanently connected"** as used in this description is intended to mean that the parts, which in this application is embodied as a cartridge and a needle assembly, requires the use of tools in order to be separated and should the parts be separated it would permanently damage at least one of the parts.

All references, including publications, patent applications, and patents, cited herein are incorporated by reference in their entirety and to the same extent as if each reference were individually and specifically indicated to be incorporated by reference and were set forth in its entirety herein.

All headings and sub-headings are used herein for convenience only and should not be constructed as limiting the invention in any way.

The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention. The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

### BRIEF DESCRIPTION OF THE DRAWINGS:

Figure 1 show in part a cross-sectional view of a first embodiment of an injection device with the protective cap assembly removed.
Figure 2 show in part a cross-sectional view of the first embodiment of the injection device with the protective cap assembly mounted.
Figure 3 show a cross-sectional view of the protective cap assembly according to the first embodiment of the invention.
Figure 4 show a perspective view of the inner sealing element of figure 3.
Figure 5 show a cross-sectional view of the inner sealing element of figure 3.
Figure 6 show a different embodiment in which a one-way valve is established.
Figure 7 show in part a cross-sectional view of a further embodiment of an injection device without the needle assembly mounted.
Figure 8 show in part a cross-sectional view of the injection device of figure 7 with the needle assembly mounted.
Figure 9 show in part a cross-sectional view of the injection device of figure 7 with both the needle assembly and the protective cap assembly mounted.
Figure 10 show a cross-sectional view along the line A-A in figure 8.
Figure 11 show an expanded view of the hashed box of Figure 9.
Figure 12 show details of an exemplary embodiment of the piston rod foot and the piston rod of the figures 6 to 9.

### DETAILED DESCRIPTION OF EMBODIMENTS:

Various aspects and embodiments of an injection device for delivering set doses of a liquid drug as disclosed herein will now be described with reference to the figures.

When relative expressions such as "upper" and "lower", "right" and "left", "horizontal" and "vertical", "clockwise" and "counter clockwise" or similar are used in the following terms, these only refer to the appended figures and not to an actual situation of use. The shown figures are schematic representations for which reason the configuration of the different structures as well as their relative dimensions are intended to serve illustrative purposes only.

In that context it may be convenient to define that the term "distal end" in the appended figures is meant to refer to the end of the injection device which usually carries the injection needle whereas the term "proximal end" is meant to refer to the opposite end pointing away from the injection needle.

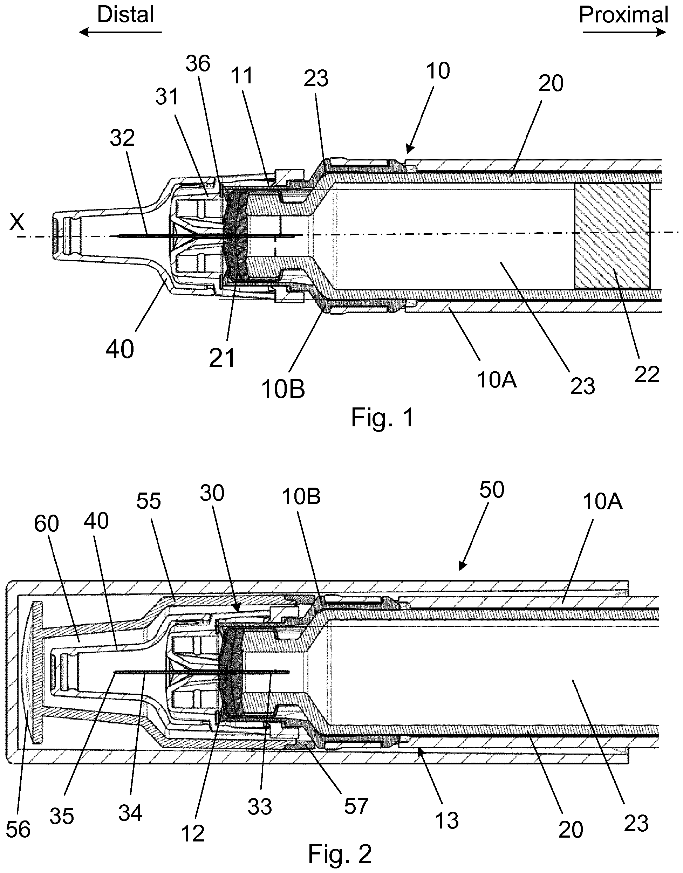

Distal and proximal are meant to be along an axial orientation extending along the longitudinal axis "X" and is further disclosed in figure 1.

Some of the different components are only disclosed in relation to a single embodiment of the invention, but is meant to be included in the other embodiments without further explanation.

A partial view of an injection device according to a first embodiment of the present invention is disclosed in figure 1. The outer shell is formed from a housing assembly 10 which can be made from several parts. In the disclosed embodiment the housing assembly 10 comprises a main housing part 10A and a distal housing part 10B. The distal housing part 10B is click fitted to the main housing part 10A but could alternatively be moulded as one unit.

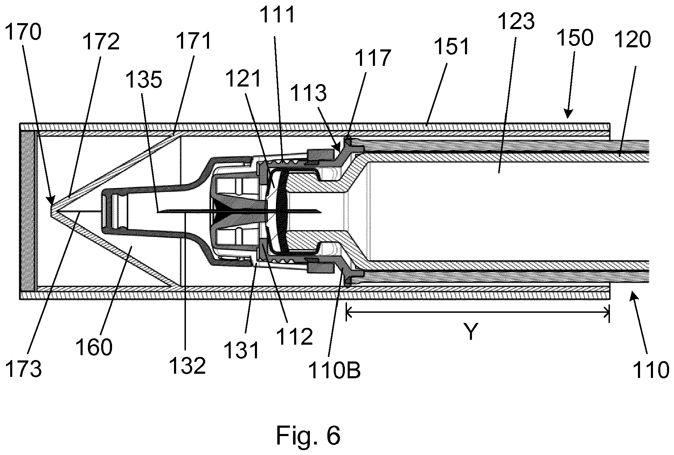

The distal housing part 10B further carries a needle mount 11 which in figure 1 and in figure 2 is depicted as being a bayonet interface and in figure 6 is depicted (111) as being a threaded interface. Both a bayonet interface and a threaded interface are well-known in the prior art of needle interfaces for injection devices.

The housing assembly 10 holds a cartridge 20 which cartridge 20 distally is sealed by a pierceable septum 21 and proximally closed by a movable plunger 22, which together with the cartridge 20 defines an interior space 23 containing the liquid drug to be injected. The cartridge 20 is distally provided with a so called neck which has a shoulder 23 which is supported by the distal housing part 10B as disclosed in figure 1.

Further, the distal housing part 10B terminates in a radially pointing flange 12 which in the disclosed embodiment is inclined in the proximal direction. This radial flange 12 proximally seals against the distal end of the cartridge 20. The ratio between the radial flange 12 and the shoulder 23 of the cartridge 20 is such that the distal end of the cartridge 20 is urged against the flange 12

The cartridge 20 can either be permanently embedded in the housing assembly 10 if the injection device is a so-called pre-filled injection device, or the cartridge 20 can be exchangeable if the injection device is a so-called durable injection device. However, in either type of injection devices a surface structure or the like can be designed such that it applies an axial pressure on the cartridge 20 in the distal direction. This pressure secures that the seal between the distal end of the cartridge 20 and the radial flange 12 of the housing assembly 10 is tight.

As disclosed in figure 1 and 2, the needle mount 11 carries a needle assembly 30 which comprises a needle hub 31 to which a needle cannula 32 is secured. Such needle assemblies 30 are often referred to as pen-needles. The needle cannula 32 has a proximal end 33 which, when the needle assembly 30 is attached to the needle mount 11 penetrates through the pierceable septum 21 and into the interior 23 of the cartridge 20 such that liquid drug is free to flow through the longitudinal lumen of the needle cannula 32 during injection. The needle cannula 32 further has a distal end 34 laying distal to the hub 31 and terminating in a distal tip 35 which during injection penetrates the skin of the user.

The needle hub 31 is further provided with a radial wall 36 on its inner surface which axially abuts the distal surface of the radial flange 12 of the housing assembly 10 as depicted in figure 1 and 2. The needle mount 11, being threaded or a bayonet can be formed such that the radial wall 36 is pulled against the radial flange 12 as the needle hub 31 is secured to the needle mount 11.

The seal between this radial wall section 36 of the needle hub 31 and the distal surface of the radial flange 12, and the seal between the distal end of the cartridge 20 and the proximal surface of the radial flange 12 of the of the housing assembly 10 assures that no or only a very limited amount of air-flow is possible into the air-tight chamber 60. The abutment provided between the shoulder 23 of the cartridge 20 and the housing assembly 10 also reduces this air-flow.

Distally the needle hub 31 carries an inner hut 40 which is press fitted to an outer surface of the hub 31. The inner hut 40 can be easily removed by the user prior to performing an injection and is designed to protect the user from being unintentional injured by the sharp distal tip 35 of the needle cannula 32. After an injection has been performed the user can re-mount the inner hut 40 if so desired.

Once an injection has been performed, the user usually re-mounts the protective cap assembly 50 as disclosed in figure 2. This protective cap assembly 50 protects the distal end of the injection device between injections and is press fitted onto the distal end of the housing assembly 10 and is easy removable by the user. The inner surface 52 of the protective cap assembly 50 can also be provided with active engaging means engaging similar receiving parts provided on the outer surface 13 of the housing assembly 10. Such arrangement could e.g. be a bayonet interface. The protective cap assembly 50 is in the disclosed embodiment designed to only cover the distal end of the housing assembly 10 of the injection device but can have any desired length. The protective cap assembly 50 can further be mounted both with the needle assembly 30 attached as disclosed in figure 2 and with the needle assembly 30 removed. The protective cap assembly 50 is further designed such that it has sufficient space for the inner hut 40 should the user decide also the re-mount the inner hut 40 after an injection has been performed. The protective cap assembly 50 is obviously removed prior to performing an injection.

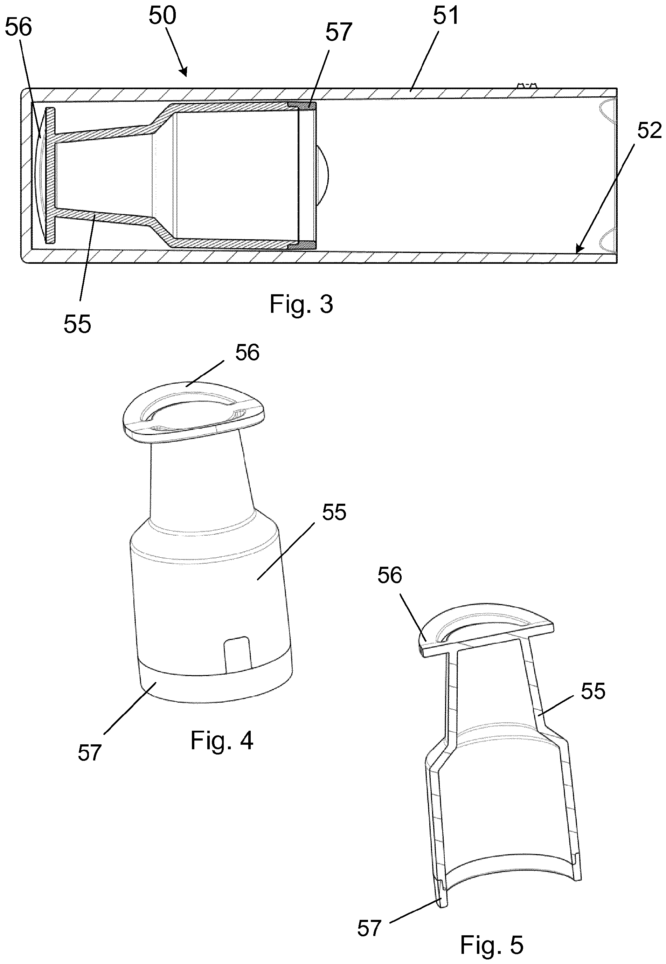

The protective cap assembly 50 is disclosed in a cross sectional view in figure 3 and comprises two parts; an outer cap element 51 and an inner sealing element 55.

The inner sealing element 55 is further disclosed in details in figure 4 and 5. Distally the inner sealing element 55 carries a resilient element 56 which in the disclosed embodiment is moulded as an integral part of the inner sealing element 55. However, this resilient element 56 could be any kind of separate compression spring interacting between the outer cap element 51 and the inner sealing element 55.

The inner sealing element 55 is proximally provided with an air-tight seal 57 moulded from a rubber material such as a thermoplastic elastomer. The inner sealing element 55 and the air-tight seal 57 is preferably formed by using a well-known 2K moulding technique, but the air-tight seal 57 could also be a separate seal 57 which is e.g. glued or welded to the inner sealing element 55.

The inner sealing element 55 is mounted inside the outer cap element 51 such that the resilient element 56 urges the inner sealing element 55 in the proximal direction relatively to the outer cap element 51 as depicted in figure 3.

When the user re-mounts the protective cap assembly 50 to the distal end of the housing assembly 10 following an injection, the air-tight seal 57 will abut the housing assembly 10 as disclosed in figure 2 and thus form an air-tight chamber 60 in which the distal tip 35 of the needle cannula 32 lies between injections. Further the resilient element 56 will urge the air-tight seal 57 against the housing assembly 10 such that the air-tight seal 57 is pressurized.

By being pressurized is meant that the air-tight seal 57 is pushed against the housing assembly 10 with an active force. In the disclosed embodiment in the figures 1 to 5, the air-tight seal 57 further abuts an inclined surface on the distal housing part 10B to further enhance the abutment.

If the needle assembly 30 is maintained attached to the distal housing part 10B between injections an air-tight chamber 60 is established between the sealing element 55 and the housing assembly 10 and as the distal tip 35 of the needle cannula 32 is maintained inside this air-tight chamber 60 between subsequent injections, the liquid drug present in the longitudinal lumen of the needle cannula 32 is prevented from drying out.

This is achieved since the air-tight chamber 60, when being heated or just warm, around the distal tip 35 of the needle cannula 32 quickly will be humidified by evaporating liquid from the cartridge 20, whereby a relatively high humidity around the distal tip 35 of the needle cannula 32 actively will prevent clogging.

Figure 6 discloses a different embodiment in which the same elements are numbered using the same numbers as in the first embodiment however with a "1" in front. The needle mount which in figure 3 is disclosed as a threaded interface is thus numbered 111 and the needle hub 131.

As can be seen from figure 6, the air-tight seal 117 is in this second embodiment provided between the distal housing part 110B and the protective cap assembly 150. In the first embodiment the air-tight seal 57 were moulded integrally with the protective cap assembly 150, however in this second embodiment, the air-tight seal 117 is preferably moulded as an integral part of the distal housing part 110B using a 2K moulding technique.

In this embodiment, the distal end of the cartridge 120 and the needle hub 131 also abut and seals against either surface of the radial flange 112 of the housing assembly 110 which radial flange 112 in this particular embodiment is disclosed as not being inclined.

When the user re-mounts the protective cap assembly 150 to the housing assembly 110 the protective cap assembly 150 slides the distance indicated with an "Y" in figure 6. Since the protective cap assembly 150 slides relatively to the air-tight sealing 117, the air trapped inside the protective cap assembly 150 will be compressed. Due to this compression there is a risk that this trapped air will be pressed through the longitudinal lumen of the needle cannula 132 and into the interior 123 of cartridge 120 which is unwanted since it creates air bubbles in the liquid drug.

To prevent this and to allow the trapped air to escape, the interior of the protective cap assembly 150 is provided with a one-way valve 170 which can be embodied in a number of different ways. This one-way valve 170 is in the disclosed embodiment formed by a rubber insert 171 moulded on the inside of the outer cap element 151 and which rubber insert 171 distally terminates in a closed tip 172 provided with a longitudinal slit 173. Thus when the protective cap assembly 150 slides the distance "Y" proximally on the housing assembly 110 the trapped air can escape through this longitudinal slit 173.

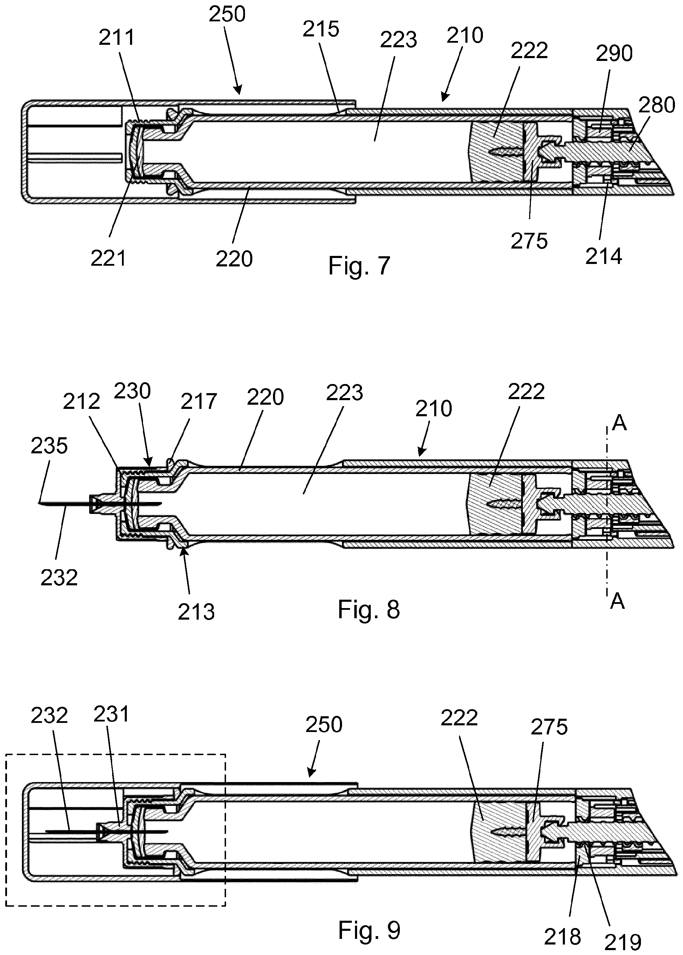

The figures 7 to 12 disclose a third embodiment wherein the same elements are numbered as in the previous two embodiments however with an "2" in front of the number. The housing assembly is thus numbered 210 and the protective cap assembly is numbered 220.

The previous described one-way valve 170 can also be used as a part of this third embodiment without further explanation.

In figure 7 to 9 the distal end of the housing assembly 210 is formed as one structural unit as the distal housing part and the main housing part is moulded as one unitary housing. The distal end of the housing assembly 210 is also in this embodiment provided with a needle mount 211 which is depicted as being a threaded interface.

The housing assembly 210 stores the cartridge 220 which cartridge 220 is mounted in the main housing or in a cartridge holder connected to the main housing distally to a piston rod 280 as it is generally known from various types of injection devices.

The housing assembly 210 has an outer surface as indicated by arrow 213 in Figure 1 and is, as in the previous embodiments, provided with a radial flange 212 which distally terminates the housing assembly 220 and seals both against the needle hub 231 and the cartridge 220.

The housing 210 further comprises a nut element 218 or similar for engaging with the piston rod 280. In the disclosed and similar embodiments, the nut element 218 is inrotatable connected to the housing assembly 210 or e.g. moulded as a part of the housing assembly 210. The nut element 218 has an internal thread 219 which threadely engages with an outer thread 281 provided externally on the piston rod 280 such that the piston rod 280 is moved helically when rotated relatively to the housing assembly 210 and thus to the nut element 218. In the disclosed embodiment, the nut element 218 is externally provided with a plurality of external teeth engaging the housing assembly 210 and maintaining the nut element 218 inrotatable in relation to the housing assembly 210.

The cartridge 220 has as in the previous embodiments a distal end being closed by a pierceable septum 221 or the like and a proximal end being closed by a movable plunger 222 defining an interior 223 containing a liquid drug to be expelled during use. As shown, the pierceable septum 221 comprises a double membrane but it could also be different, e.g. comprising only a single membrane.

As disclosed in figure 8, a needle assembly 230 can be mounted onto the needle mount 211. This needle assembly 230 comprises a needle cannula 232 being connected to the needle hub 231 as in the previous embodiments. The needle cannula 232 has a distal end 234 with a distal tip 235 and a proximal end 233 that, when the needle assembly 230 is properly attached to the injection device, is in liquid communication with the interior 223 of the cartridge 220.

The injection device also comprises the protective cap assembly 250 surrounding at least the distal end of the housing assembly 210 when the protective cap assembly 250 is fitted to the housing assembly 210 as depicted in figure 7 and in figure 9. As can be seen e.g. in figure 7, the housing assembly 210 is provided with a longitudinal window 215 through which the user can inspect the liquid drug contained in the interior 223 of the cartridge 220. This longitudinal window 215 is covered by the protective cap assembly 250 between injections to shield the liquid drug from especially ultra violet light.

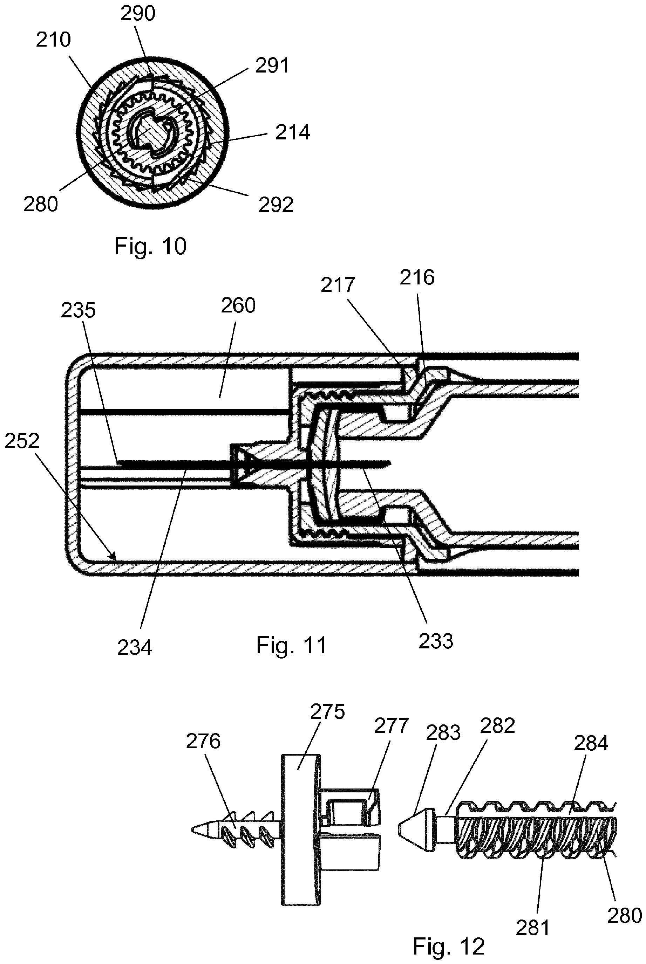

A piston rod foot 275 disclosed in details in figure 12 is provided between the movable plunger 222 of the cartridge 220 and the piston rod 280 to distribute an injection pressure over a larger surface of the movable plunger 222. In certain previous injection devices, the piston rod foot or washer is loosely disposed between the movable plunger and the piston rod. However, in the present embodiment both the movable plunger 222 and the piston rod 280 are secured to the piston rod foot 275. As a result of this permanent connection the piston rod 280 follows axial movement of the movable plunger 222 caused by the liquid drug expanding or contracting due to temperature changes. However, in order for the piston rod 280 to move freely due to temperature changes occurring during storage, the piston rod 280 needs to be decoupled from the drive system during storage.

The piston rod foot 275 may be rotatable hinged to the piston rod 280 or may be moulded integrally with the piston rod 280 to form one stiff construction.

In the embodiment shown in the figures 7 to 9, the piston rod foot 275 is fully and not only axially fixed to the movable plunger 222, i.e. they cannot move axially nor rotate in relation to each other. This is in one example (see e.g. figure 12) done by providing the piston rod foot 275 with a spike 276 on its distally pointing side which spike 276 is rammed into the movable plunger 222. Proximally, the piston rod foot 275 is provided with a number of flexible arms 277 which snaps together with a track 282 provided distally at the piston rod 280 such that the piston rod 280 and the piston rod foot 275 are axially locked but able to rotate in relation to each other. Distally to the track 282, the piston rod 280 can be provided with an inclined distal end 283 that presses the piston rod foot 275 forward when the piston rod 280 and piston rod foot 275 are connected to thereby reduce or preferably eliminate any play between the piston rod 280 and the piston rod foot 275.

Further details of this arrangement are e.g. disclosed in patent publication WO 2014/060369 which also discloses how the drive mechanism can be designed such that the piston rod 280 default is released from the drive mechanism during storage and only coupled to the drive mechanism during dose expelling. This means that the piston rod 280 initially is free to rotate unless the user is actually expelling a dose.

According to an aspect of the present invention, the housing assembly 210 - as in the second embodiment shown in figure 6 - comprises an air-tight seal 217 located on and all the way around the external surface 213 of the housing assembly 210, e.g. towards the distal end of the housing assembly 210 in the proximity of the needle mount 211 to which the needle hub 231 is attached.

In this embodiments, the air-tight seal 217 is provided between an inner surface 252 of the cap assembly 250, when fitted to the housing assembly 210, and the external surface 213 of the housing assembly 210 such that an air-tight chamber 260 is established surrounding at least the distal tip 235 of the distal end of the needle cannula 230.

If needed be, an additional air-tight seal 216 (see figure 11 which is an enlarged view of the hashed box in figure 9) can be located between an internal surface of the housing assembly 210 and the shoulder 223 of the cartridge 220. This additional air-tight seal 216 is preferably moulded as a part of the housing assembly 210. However, the seal between the radial flange 212 and the distal end of the cartridge 220 and the needle hub 231 is usually sufficient to maintain the air-tight chamber 260 in fact air-tight.

When the injection device according to the third embodiment (with or without the additional air-tight seal 216) is fitted with the protective cap assembly 250 the air-tight chamber 260 is established around the distal tip 235 of the needle cannula 107 as disclosed in figure 9.

Should the injection device with a needle assembly 230 mounted under the protective cap assembly 250 be subject to a temperature decrease, the air inside the sealed air-tight chamber 260 will contract and more importantly the air, being a gas, will contract more than what the liquid drug, obviously being a liquid, in the interior 223 of the cartridge 220 will do. This creates an under-pressure in the air- tight chamber 260 that therefore will draw out a small portion of the liquid drug out of the cartridge 220 through the lumen of the needle cannula 232. Additionally, there will be a higher flow out of the needle cannula 232 than the contraction of volume of the liquid drug inside the cartridge 220, which will ensure that the flow of liquid out will continue for as long as both the air and the liquid are contracting thereby efficiently ensuring that air will not enter into the liquid drug contained in the interior 223 in the cartridge 220.

In this way, it is efficiently avoided that air bubbles are introduced into the liquid drug inside the cartridge 220 or at least the likelihood thereof is reduced.

If the movable plunger 222, the piston rod foot 275, and the piston rod 280 are axially connected as described above, the under-pressure in the air-tight chamber 260 and the smaller contraction of the liquid drug will move the movable plunger 222 towards the distal end of the cartridge 220 and with it also the piston rod foot 275 and the piston rod 280. It is however noted, that air bubbles will still be avoided or reduced even if these elements are not axially connected.

According to a further example of the injection device where the movable plunger 222, the piston rod foot 275, and the piston rod 280 rod are axially connected, the piston rod 280 is only able to move in the distal direction due to the presence of a one-way mechanism as will be explained.

When the temperature increases, both the air in the air-tight chamber 260 and the liquid drug inside the cartridge 220 will expand, however, the air will expand the most creating an over pressure that would push the movable plunger 222 in the proximal direction potentially pumping or pressing air into the interior 223 of the cartridge 220 thereby introducing air bubbles in the liquid drug.

However, this is avoided or at least reduced to a great extent by the movable plunger 222, the piston rod foot 275, and the piston rod 280 being axially connected and arranged to only move axially in the distal direction. Thereby air bubbles are not introduced into the liquid drug due to being exposed to a higher temperature.

According to this example, the one-way mechanism comprises a ratchet element 290 that limits the movement of the piston rod 280 to be possible only in the distal direction in a simple way. The ratchet element 290 internally has a key shape 291 which fits in a longitudinal track 284 provided in the piston rod 280 such that the ratchet element 290 and the piston rod 280 rotate in unison. The ratchet element 290 could thereby also works as a drive element driving the piston rod 280 through rotation. The ratchet element 290 further comprises one or more resilient arms 292 which engage a toothed ring 214 provided internally in the housing assembly 210 such that the ratchet element 290 can only rotate in one rotational direction. The allowed rotational direction being the direction that moves the piston rod 280 forward in the distal direction. The movable plunger 222 is thereby prevented from moving proximally.

In the example in which the ratchet element 290 also works as the drive element any rotation of the ratchet element 290 also rotates the piston rod 280 to rotate helically in the thread 219 of the nut element 218.. However, during storage the ratchet element 290 would be decoupled from the drive mechanism such that the ratchet element 290 and thus the piston rod 280 is able to rotate freely. The rotation of the ratchet element 290 is however limited to one rotational direction and preferably the direction which moves the piston rod 280 in the distal direction.

In other examples, the air-tight seal 217 has a first position and a second position. In the first position disclosed in figure 7, the air-tight chamber 260 is not established and the air-tight seal 217 will not be in contact with the inner surface 252 of the cap assembly 250. In the second position disclosed in figure 9, the air-tight chamber 260 is established and the air-tight seal 217 will be in contact with the inner surface 252 of the cap assembly 250.

This has the advantage that the air-tight seal 217 may be in its first, e.g. rested, relaxed, or non-active, position e.g. during storage at the manufacturer or the user until use. In this way, relaxation of the air-tight seal 217, e.g. when being a rubber seal or the like, is prevented even when being stored for a relatively long time.

In at least some of the embodiments, the air-tight seal 217 is brought from the first position to the second position in response to the needle assembly 230 being attached to the injection device 100. Figure 8 discloses that the needle hub 231 engages the air-tight seal 217 and applies a pressure in the proximal direction which radially expand the air-tight seal 217 to engage with the inner surface 252 of the protective cap assembly 250 as depicted in figure 9.

This activates the use or function of the air-tight seal 217 at the specific time when it is needed in a very user convenient and expedient manner simply by attaching the needle assembly 230 to the needle mount 211 of the housing assembly 210, as the user would do any way before regular use. Alternatively, other ways of switching between or providing the first and second position may be used.

Some preferred embodiments have been shown in the foregoing, but it should be stressed that the invention is not limited to these, but may be embodied in other ways within the subject matter defined in the following claims.

In the claims enumerating several features, some or all of these features may be embodied by one and the same element, component or item. The mere fact that certain measures are recited in mutually different dependent claims or described in different embodiments does not indicate that a combination of these measures cannot be used to advantage.

## Claims

1. An injection device for injecting a plurality of doses of a liquid drug through a number of injections, the injection device comprising:
- a housing assembly (10, 110, 210) supporting a cartridge (20, 120, 220), at least in use, which cartridge (20, 120, 220) has a distal end sealed by a pierceable septum (21, 121, 221) and a proximal end sealed by a movable plunger (22, 222) defining an interior (23, 123, 223) containing the liquid drug,
- a piston rod (280) for moving the movable plunger (22, 220) inside the cartridge (20, 120, 220),
- a removable protective cap assembly (50, 150, 250) removable coupled to the housing assembly (10, 110, 210) and surrounding at least a distal end of the housing assembly (10, 110, 210) between injections, and
- a needle mount (11, 111, 211) securing a cannula (32, 132, 232), at least in use, which needle cannula (11, 111, 211) has a proximal end (33, 233) in liquid communication with the interior (23, 123, 223) of the cartridge (20, 120, 220) and a distal end (34, 234) with a distal tip (35, 135, 235) for penetrating the skin of a user, and wherein
the removable protective cap assembly (50, 150, 250) surrounds at least the distal tip (35, 235) of the needle cannula (32, 132, 232) between injections, and an air-tight seal (57, 117, 217) is operational provided between the removable protective cap assembly (50, 150, 250) and the housing assembly (10, 210, 210) or a needle hub (31, 131, 231) mounted to the needle mount (11,111,211) **characterised in that** an air-tight chamber (60, 160, 260) is established surrounding at least the distal tip (35, 135, 235) of the needle cannula (32, 132, 232) when the removable protective cap assembly (50, 150, 250) is fitted to the distal end of the housing assembly (10, 110, 210) between injections.

2. An injection device according to claim 1, wherein the needle cannula (32, 132, 232) is mounted in the needle hub (31, 131, 231) attachable to the needle mount (11, 111, 211) provided distally at the housing assembly (10, 110, 210).

3. An injection device according to claim 2, wherein the housing assembly (10, 110, 210) distally is provided with a radial flange (12, 112, 212) against which flange (12, 112, 212) both the needle hub (31, 131, 231) and the cartridge (20, 120, 220) abuts.

4. An injection device according to any of the claims 1 to 3, wherein the air-tight seal (57) is provided on an inner surface (52) of the removable protective cap assembly (50).

5. An injection device according to any of the claims 1 to 4, wherein the removable protective cap assembly (50) comprises an inner sealing element (55) and an outer cap element (51).

6. An injection device according to claim 5, wherein the inner sealing element (55) carries the air-tight seal (57).

7. An injection device according to claim 5 or 6, wherein the inner sealing element (55) is coupled to the outer cap element (51) via a resiliency such as a resilient element (56) providing the sealing element (55) with an axial force relatively to the outer cap element (51).

8. An injection device according to any of the claims 1 to 3, wherein the air-tight seal (117, 217) is provided on an external surface (113, 213) of the housing assembly (110, 210) preferably in the proximity of the needle mount (111, 211).

9. An injection device according to claim 8, wherein the air-tight seal (117, 217) is radially expandable to cover a larger outer diameter when the needle hub (131, 231) is attached to the needle mount (111, 211).

10. An injection device according to any of the preceding claims, wherein the injection device further comprises a ratchet element (290) limiting axial movement of the piston rod (280) to the distal direction.

11. An injection device according to any of the preceding claims, wherein the piston rod (280) and the movable plunger (22, 122, 222) are axially coupled together.

12. An injection device according to claim 11, wherein the piston rod (280) and the movable plunger (22, 122, 222) are axially coupled via an intermediate piston rod foot (275) which is fixed to the movable plunger (22, 122, 222) and rotatably connected with the piston rod (280).

13. An injection device according to any of the preceding claims, wherein the injection device further comprises an additional air-tight seal (216) located between an internal surface of the housing assembly (10, 210, 210) and an outer surface of the cartridge (20, 120, 220).

14. An injection device according to any of the preceding claims, wherein the protective cap assembly (50, 150, 250) is provided with a one-way valve (270).

## Patentansprüche

1. Injektionsvorrichtung zum Injizieren mehrerer Dosen eines flüssigen Medikaments über eine Reihe von Injektionen, die Injektionsvorrichtung umfassend:
- eine Gehäuseanordnung (10, 110, 210), die, zumindest im Gebrauch, eine Patrone (20, 120, 220) hält, wobei die Patrone (20, 120, 220) ein distales Ende, das durch ein durchstechbares Septum (21, 121, 221) verschlossen ist, und ein proximales Ende, das durch einen beweglichen Kolben (22, 222), der einen Innenraum (23, 123, 223) definiert, der das flüssige Medikament enthält, verschlossen ist, aufweist,
- eine Kolbenstange (280) zum Bewegen des beweglichen Kolbens (22, 220) im Inneren der Patrone (20, 120, 220),
- eine abnehmbare Schutzkappenanordnung (50, 150, 250), die mit der Gehäuseanordnung (10, 110, 210) abnehmbar verbunden ist und zwischen Injektionen zumindest ein distales Ende der Gehäuseanordnung (10, 110, 210) umgibt, und
- eine Nadelhalterung (11, 111, 211), die, zumindest im Gebrauch, eine Kanüle (32, 132, 232) festhält, wobei die Nadelkanüle (11, 111, 211) ein proximales Ende (33, 233) in Flüssigkeitsverbindung mit dem Innenraum (23, 123, 223) der Patrone (20, 120, 220) und ein distales Ende (34, 234) mit einer distalen Spitze (35, 135, 235) zum Eindringen in die Haut eines Benutzers aufweist, und wobei
die abnehmbare Schutzkappenanordnung (50, 150, 250) zwischen Injektionen mindestens die distale Spitze (35, 235) der Nadelkanüle (32, 132, 232) umgibt und eine luftdichte Abdichtung (57, 117, 217) betriebsmäßig zwischen der abnehmbaren Schutzkappenanordnung (50, 150, 250) und der Gehäuseanordnung (10, 210, 210) oder einer Nadelmuffe (31, 131, 231), die an der Nadelhalterung (11, 111, 212) befestigt ist, bereitgestellt ist, **dadurch gekennzeichnet, dass** eine luftdichte Kammer (60, 160, 260) geschaffen wird, die mindestens die distale Spitze (35, 135, 235) der Nadelkanüle (32, 132, 232) umgibt, wenn die abnehmbare Schutzkappenanordnung (50, 150, 250) zwischen Injektionen am distalen Ende der Gehäuseanordnung (10, 110, 210) angebracht ist.

2. Injektionsvorrichtung nach Anspruch 1, wobei die Nadelkanüle (32, 132, 232) in der Nadelmuffe (31, 131, 231) befestigt ist, die an der Nadelhalterung (11, 111, 211), die an der Gehäuseanordnung (10, 110, 210) distal bereitgestellt ist, angebracht werden kann.

3. Injektionsvorrichtung nach Anspruch 2, wobei die Gehäuseanordnung (10, 110, 210) distal mit einem Radialflansch (12, 112, 212) versehen ist, wobei sowohl die Nadelmuffe (31, 131, 231) als auch die Patrone (20, 120, 220) an dem Flansch (12, 112, 212) anliegt.

4. Injektionsvorrichtung nach einem der Ansprüche 1 bis 3, wobei die luftdichte Abdichtung (57) an einer Innenfläche (52) der abnehmbaren Schutzkappenanordnung (50) bereitgestellt ist.

5. Injektionsvorrichtung nach einem der Ansprüche 1 bis 4, wobei die abnehmbare Schutzkappenanordnung (50) ein inneres Dichtungselement (55) und ein äußeres Kappenelement (51) umfasst.

6. Injektionsvorrichtung nach Anspruch 5, wobei das innere Dichtungselement (55) die luftdichte Abdichtung (57) aufweist.

7. Injektionsvorrichtung nach Anspruch 5 oder 6, wobei das innere Dichtungselement (55) mit dem äußeren Kappenelement (51) mittels Elastizität verbunden ist, etwa durch ein elastisches Element (56), das das Dichtungselement (55) mit einer Axialkraft in Bezug auf das äußere Kappenelement (51) versieht.

8. Injektionsvorrichtung nach einem der Ansprüche 1 bis 3, wobei die luftdichte Abdichtung (117, 217) an einer Außenfläche (113, 213) der Gehäuseanordnung (110, 210), vorzugsweise in der Nähe der Nadelhalterung (111, 211) bereitgestellt ist.

9. Injektionsvorrichtung nach Anspruch 8, wobei die luftdichte Abdichtung (117, 217) radial erweiterbar ist, um einen größeren Außendurchmesser abzudecken, wenn die Nadelmuffe (131, 231) an der Nadelhalterung (111, 211) befestigt ist.

10. Injektionsvorrichtung nach einem der vorhergehenden Ansprüche, wobei die Injektionsvorrichtung ferner ein Sperrglied (290) umfasst, das eine axiale Bewegung der Kolbenstange (280) in die distale Richtung begrenzt.

11. Injektionsvorrichtung nach einem der vorhergehenden Ansprüche, wobei die Kolbenstange (280) und der bewegliche Kolben (22, 122, 222) miteinander axial gekoppelt sind.

12. Injektionsvorrichtung nach Anspruch 11, wobei die Kolbenstange (280) und der bewegliche Kolben (22, 122, 222) über einen dazwischenliegenden Kolbenstangenfuß (275) axial gekoppelt sind, der an dem beweglichen Kolben (22, 122, 222) befestigt und mit der Kolbenstange (280) drehbar verbunden ist.

13. Injektionsvorrichtung nach einem der vorhergehenden Ansprüche, wobei die Injektionsvorrichtung ferner eine zusätzliche luftdichte Abdichtung (216) umfasst, die sich zwischen einer Innenfläche der Gehäuseanordnung (10, 210, 210) und einer Außenfläche der Patrone (20, 120, 220) befindet.

14. Injektionsvorrichtung nach einem der vorhergehenden Ansprüche, wobei die Schutzkappenanordnung (50, 150, 250) mit einem Einwegventil (270) versehen ist.

## Revendications

1. Dispositif d'injection pour l'injection d'une pluralité de doses d'un médicament liquide en plusieurs injections, le dispositif d'injection comprenant :
- un ensemble boîtier (10, 110, 210) supportant au moins une cartouche (20, 120, 220), en utilisation, laquelle cartouche (20, 120, 220) a une extrémité distale scellée par un septum perforable (21, 121, 221) et une extrémité proximale scellée par un piston mobile (22, 222) définissant un intérieur (23, 123, 223) contenant le médicament liquide,
- une tige de piston (280) pour le déplacement du piston mobile (22, 220) à l'intérieur de la cartouche (20, 120, 220),
- un ensemble capuchon de protection amovible (50, 150, 250) couplé de manière amovible à l'ensemble boîtier (10, 110, 210) et entourant au moins une extrémité distale de l'ensemble boîtier (10, 110, 210) entre les injections, et
- un support d'aiguille (11, 111, 211) de fixation d'au moins une canule (32, 132, 232), en utilisation, laquelle canule à aiguille (11, 111, 211) a une extrémité proximale (33, 233) en communication liquide avec l'intérieur (23, 123, 223) de la cartouche (20, 120, 220) et une extrémité distale (34, 234) avec une pointe distale (35, 135, 235) pour la pénétration dans la peau d'un utilisateur, et dans lequel
l'ensemble capuchon de protection amovible (50, 150, 250) entoure au moins la pointe distale (35, 235) de la canule à aiguille (32, 132, 232) entre les injections et un joint étanche à l'air (57, 117, 217) est fourni de manière opérationnelle entre l'ensemble capuchon de protection amovible (50, 150, 250) et l'ensemble boîtier (10, 210, 210) ou un raccord d'aiguille (31, 131, 231) monté sur le support d'aiguille (11, 111, 211) **caractérisé en ce qu'**une chambre étanche à l'air (60, 160, 260) est établie entourant au moins la pointe distale (35, 135, 235) de la canule à aiguille (32, 132, 232) lorsque l'ensemble capuchon de protection amovible (50, 150, 250) est monté sur l'extrémité distale de l'ensemble boîtier (10, 110, 210) entre les injections.

2. Dispositif d'injection selon la revendication 1, dans lequel la canule à aiguille (32, 132, 232) est montée dans un raccord d'aiguille (31, 131, 231) pouvant être attaché au support d'aiguille (11, 111, 211) fourni de manière distale sur l'ensemble boîtier (10, 110, 210).

3. Dispositif d'injection selon la revendication 2, dans lequel l'ensemble boîtier (10, 110, 210) est fourni de manière distale d'un rebord radial (12, 112, 212) contre lequel rebord (12, 112, 212) à la fois le raccord d'aiguille (31, 131, 231) et la cartouche (20, 120, 220) sont en butée.

4. Dispositif d'injection selon l'une quelconque des revendications 1 à 3, dans lequel le joint étanche à l'air (57) est fourni sur une surface interne (52) de l'ensemble capuchon de protection amovible (50).

5. Dispositif d'injection selon l'une quelconque des revendications 1 à 4, dans lequel l'ensemble capuchon de protection amovible (50) comprend un élément d'étanchéité interne (55) et un élément capuchon externe (51).

6. Dispositif d'injection selon la revendication 5, dans lequel l'élément d'étanchéité interne (55) porte le joint étanche à l'air (57).

7. Dispositif d'injection selon la revendication 5 ou 6, dans lequel l'élément d'étanchéité interne (55) est couplé à l'élément de capuchon externe (51) via une résilience telle qu'un élément résilient (56) fournissant sur l'élément d'étanchéité (55) une force axiale par rapport à l'élément capuchon extérieur (51).

8. Dispositif d'injection selon l'une quelconque des revendications 1 à 3, dans lequel le joint étanche à l'air (117, 217) est fourni sur une surface externe (113, 213) de l'ensemble boîtier (110, 210), de préférence à proximité du support d'aiguille (111, 211).

9. Dispositif d'injection selon la revendication 8, dans lequel le joint étanche à l'air (117, 217) est dilatable radialement pour couvrir un diamètre extérieur plus grand lorsque le raccord d'aiguille (131, 231) est fixé au support d'aiguille (111, 211).

10. Dispositif d'injection selon l'une quelconque des revendications précédentes, dans lequel le dispositif d'injection comprend en outre un élément à rochet (290) de limitation du mouvement axial de la tige de piston (280) dans la direction distale.

11. Dispositif d'injection selon l'une quelconque des revendications précédentes, dans lequel la tige de piston (280) et le piston mobile (22, 122, 222) sont couplés axialement ensemble.

12. Dispositif d'injection selon la revendication 11, dans lequel la tige de piston (280) et le piston mobile (22, 122, 222) sont couplés axialement via un pied de tige de piston intermédiaire (275) qui est fixé au piston mobile (22, 122, 222) et est connecté de manière rotative à la tige de piston (280).

13. Dispositif d'injection selon l'une quelconque des revendications précédentes, dans lequel le dispositif d'injection comprend en outre un joint étanche à l'air supplémentaire (216) situé entre une surface interne de l'ensemble boîtier (10, 210, 210) et une surface externe de la cartouche (20, 120, 220).

14. Dispositif d'injection selon l'une quelconque des revendications précédentes, dans lequel l'ensemble capuchon de protection (50, 150, 250) est fourni avec une valve à un sens (270).
